# EUROPEAN PATENT SPECIFICATION

(11) **EP 3 148 445 B1**
(45) Date of publication and mention of the grant of the patent: **13.03.2019**
(21) Application number: 15729611.2
(22) Date of filing: 20.05.2015
(51) Int. Cl.: A61B 10/02, A61B 17/34

(54) **LASER CUT NEEDLE CANNULA WITH INCREASED FLEXIBILITY**
LASERBEARBEITETE NADELKANÜLE MIT ERHÖHTER FLEXIBILITÄT
CANULE AIGUILLÉ FABRIQUÉ PAR LASER AYANT UNE FLEXIBILITÉ AUGMENTÉE

(30) Priority: 30.05.2014 US 201462005446 P
(43) Date of publication of application: 05.04.2017
(73) Proprietor: Cook Medical Technologies LLC, Bloomington, IN 47404 (US)
(72) Inventor: CLANCY, Michael S., Monaleen, Limerick (IE)
(74) Representative: Simpson, Tobias Rutger
(86) International application number: PCT/US2015/031785
(87) International publication number: WO 2015/183663

(56) References cited:
- WO-A1-2004/006789
- WO-A1-2012/047984
- US-A- 5 573 520
- US-A1- 2007 276 352
- US-A1- 2009 131 886
- US-A1- 2010 056 862

## Description

### TECHNICAL FIELD

Presently disclosed embodiments generally relate to endoscopic surgical devices. More particularly, the disclosed embodiments pertain to needles having flexible distal ends configured for use during minimally-invasive procedures.

### BACKGROUND

Fine needle aspiration (FNA) is a diagnostic biopsy procedure used to obtain a sample from a target site in a patient body. A fine needle (e.g., 19-gauge to 25-gauge) is directed to a target site, and suction is applied to the proximal end of a lumen of the needle to aspirate cells through its distal end. The procedure typically is far less invasive than other biopsy techniques, whether performed percutaneously (e.g., to sample a suspected breast tumor or subcutaneous lesion) or endoscopically (e.g., to sample a suspected cholangiocarcinoma via a duodenoscope). Moreover, advances in endoscopic ultrasound (EUS) technology have helped physicians and patients by providing enhanced ability of a physician to visualize a biopsy needle to obtain a sample of material from a target site without requiring an open incision or use of large-bore needles and/or laparoscopic trocars.

Current FNA techniques typically obtain only a small number of cells useful for diagnostic evaluation. As a result, this technique includes a risk of false negatives where the few cells obtained in a sample do not accurately represent the presence of a tumor or other disease condition. The small sample size may also limit the diagnostic value of the procedure if the cells obtained are sufficiently few in number or sufficiently damaged during collection that they do not enable a definitive diagnosis. Accordingly it would be advantageous to provide a needle useful for EUS and/or percutaneous FNB (fine needle biopsy) that can obtain a larger sample size (e.g., a larger number of cells in the sample or a "core" comprising intact adjacent cells held together in similar form to their native location, suitable for histological analysis) without requiring a larger-gauge needle or requiring multiple passes of the needle to reliably obtain a diagnostically efficacious sample with regard to the number and integrity of the cells in the sample.

Moreover, it would be advantageous for the needle to be constructed in a manner providing for efficient operation through an endoscope, such as a side-viewing gastric endoscope (also known as a duodenoscope), including ready navigation through the curvature(s) commonly required in using such an endoscope with a minimum of time and manipulation required. As the needle travels through the endoscope, it can be forced through different angles of curvature and thus, there is opportunity for the needle tip to buckle or kink. Moreover, large gauge needles are not flexible enough to reach difficult area of the anatomy, such as the head of the pancreas. This lack of flexibility requires smaller needles to be used, which results in smaller samples being retrieved. It would therefore be advantageous to add flexibility to, or enhance the flexibility of, the distal end of the biopsy or tissue-sampling needle. Document WO 2012/047984 A1 discloses a bone marrow harvesting device comprising a needle including a shaft body with a flexible portion. Document US 2010/0056862 A1 discloses an endoscopic needle with a helical slit with a length of about 5 mm to about 20 mm. Document WO2004/006789 discloses a biopsy cannula comprising a spiral cut portion about 2.5 mm to 5 mm long.

### BRIEF SUMMARY

Essential features of the claimed invention are set out in the independent claim. Preferable features are set out in the dependent claims. The present disclosure relates to needles having increased flexibility and methods of using the same. In one aspect, the needle comprises an elongate tubular cannula including a proximal portion, a distal portion, and a cannula wall defining a cannula lumen. The cannula lumen extends longitudinally through the elongate tubular cannula. The distal portion of the elongate tubular cannula comprises a distal end, a plurality of apertures disposed through, and along a first length of, the cannula wall in a pattern enhancing flexibility relative to a second length of the cannula wall lacking the apertures, and a sealing member disposed on the plurality of apertures.

In an additional aspect, a biopsy needle is provided comprising an elongate tubular cannula including a proximal portion, a distal portion, and a cannula wall defining a cannula lumen. The cannula lumen extends longitudinally through the elongate tubular cannula. The distal portion of the elongate tubular cannula comprises a distal end, a notch through the cannula wall and open to the cannula lumen, a plurality of apertures disposed through, and along a first length of, the cannula wall in a pattern enhancing flexibility relative to a second length of the cannula wall lacking the apertures, and a sealing member disposed on the plurality of apertures. The plurality of apertures is disposed proximal to the notch in an interrupted helical pattern.

In a further aspect, a method of tissue collection is disclosed. The method comprises a step of providing an elongate tubular needle including a proximal portion, a distal portion, and a needle wall defining a needle lumen. The needle lumen extends longitudinally through the elongate tubular needle. The distal portion comprises a distal end, a notch open into the needle lumen, wherein a distal lip defining a distal end portion of the notch comprises a proximally-facing or a distally-facing cutting edge, a plurality of apertures disposed through, and along a first length of, the needle wall in a pattern enhancing flexibility relative to a second length of the needle wall lacking the apertures, and a sealing member disposed on the plurality of apertures. The method also includes the steps of directing the distal end of the needle into a target site, applying suction to the needle lumen, and moving the needle in a manner engaging the cutting edge with the target site such that a sample from the target site is collected into the needle lumen.

The foregoing has outlined rather broadly the features and technical advantages of the present disclosure in order for the detailed description that follows to be better understood. Additional features and advantages of the disclosure will be described hereinafter that form the subject of the claims of this application. It should be appreciated by those skilled in the art that the conception and the specific embodiments disclosed may be readily utilized as a basis for modifying or designing other embodiments for carrying out the same purposes of the present disclosure. It should also be realized by those skilled in the art that such equivalent and/or modified embodiments do not depart from the spirit and scope of the disclosure as set forth in the appended claims.

### BRIEF DESCRIPTION OF THE SEVERAL VIEWS OF THE DRAWINGS

In order to assist the understanding of embodiments of the invention, reference will now be made to the appended drawings, which are not necessarily drawn to scale or proportion, and in which like reference numerals generally refer to like elements. The drawings are exemplary only, and should not be construed as limiting the invention.
FIG. 1 shows an embodiment of a biopsy needle assembly;
FIGS. 2A-2D show different views of a tissue-sampling needle device embodiment;
FIGS. 3A-3D show different views of an alternate aspect of a tissue-sampling needle device;
FIG. 4 shows a sectional view of a needle according to an aspect of the present disclosure;
FIG. 5 shows a plan view of a needle according to an aspect of the present disclosure;
FIG. 6 shows an additional embodiment of a needle according to the present disclosure; and
FIG. 7 shows a further embodiment of a needle according to the present disclosure.

### DETAILED DESCRIPTION

Various aspects or embodiments are described below with reference to the drawings in which like elements generally are referred to by like numerals. The relationship and functioning of the various elements of the embodiments may better be understood by reference to the following detailed description. However, embodiments are not limited to those illustrated in the drawings. It should be understood that the drawings are not necessarily to scale, and in certain instances details may have been omitted that are not necessary for an understanding of the embodiments disclosed herein, such as - for example - conventional fabrication and assembly.

As used herein, the term "proximal" refers to the handle-end of a device held by a user, and the term "distal" refers to the opposite end. The term "surgical visualization device" refers to endoscopes including CCD, ultrasound, fiber optic, and CMOS devices, as well as other devices used for visualizing an internal portion of a patient body such as, for example, a laparoscope or bronchoscope.

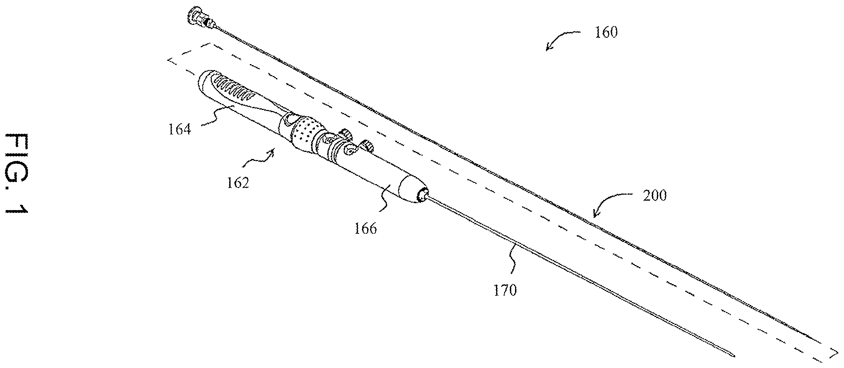

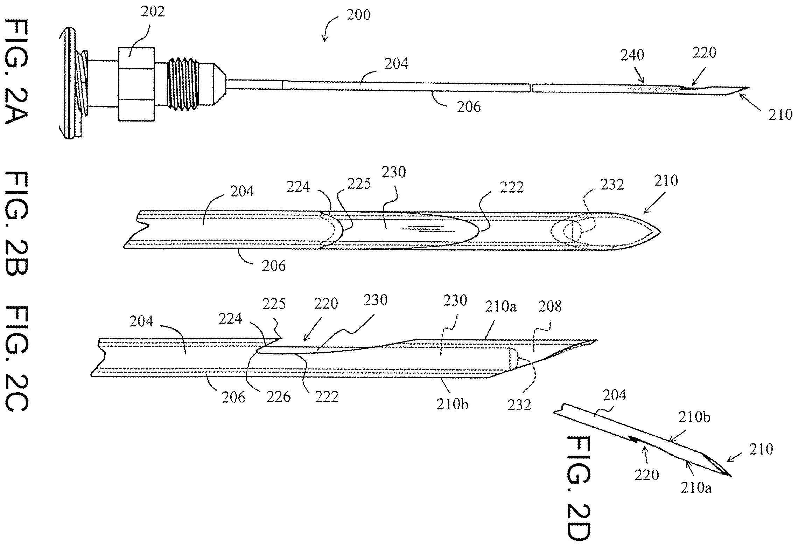

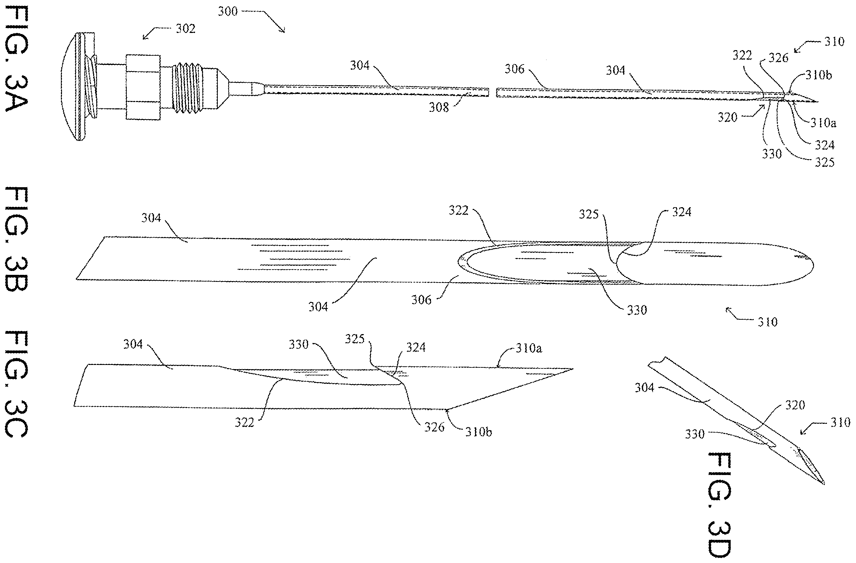

An aspect of a biopsy needle according to the present disclosure is described with reference to FIG. 1, which shows a biopsy needle assembly (160). In this aspect, the biopsy needle assembly (160) includes a handle (162). The assembly may include a sheath (170), which extends distally from the handle (162). A needle device (200) is configured to be slidably disposed longitudinally through the sheath (170), if included, and is shown and discussed below in greater detail with reference to FIGS. 2A-2D and FIGS. 3A-3D. The handle (162) may include a needle-moving element (164) and a sheath-moving element (166) in addition to lockable stops with numerical indicia for user-controlled manipulation with respect to specified distances of longitudinal movement of each. In certain aspects, the handle may be configured in a manner disclosed in U.S. Pat. No. 6,976,955 to Hardin, et al.

Detailed views of certain aspects of the needle portion of the biopsy needle assembly (160) are depicted in FIGS. 2A-2D. These figures show a biopsy needle device (200), which includes a flexible/non-rigid needle cannula (204) and a stylet (230) disposed therethrough. As shown in the side plan view of FIG. 2A, the device includes a proximal handle or hub (202) from which the elongate tubular cannula (204) extends distally. In certain embodiments, the hub (202) may be mounted or otherwise incorporated into a handle, such as the handle (162) shown in FIG. 1. The cannula (204) includes a cannula wall (206) that defines a cannula lumen (208). A distal end (210) of the cannula (204) is beveled, including a long side (210a) substantially parallel with the central longitudinal axis of the cannula (204) and extending to its distal-most tip end. A short side (210b) of the beveled distal end (210) is opposite the long end (210a).

A detailed illustration of the distal end (210) is shown in the top plan view of FIG. 2B, which, like FIGS. 2C-2D, shows only a distal end length of the device (200) shown in FIG. 2A. Other embodiments may include a double bevel, where one beveled surface is opposite the notch (220), or single or double bevels that are at least partially transverse relative to the notch (220). In some aspects, the biopsy needle device (200) does not include a notch. As will be further described below, any of the foregoing aspects of the presently disclosed biopsy needle may include one or more laser cut apertures disposed in the cannula wall near a distal portion or distal end (210) of the elongate tubular cannula. The apertures add flexibility to the needle, thereby allowing physicians to utilize larger gauge needles, obtain larger cytology or histology samples from difficult to reach areas of the anatomy, resulting in less needle passes (shorter procedures) and less traumatic procedures for the patient.

Other views of certain aspects of the needle portion of the biopsy needle assembly (160) are depicted in FIGS. 3A-3D. These figures show a biopsy needle device (300), which includes a needle cannula (304) and a stylet (330). A similar biopsy needle device can be found in International Patent Application No. PCT/US2011/31048 titled "Endoscopic Ultrasound-Guided Biopsy Needle". As shown in the side plan view of FIG. 3A, the device includes a proximal handle or hub (302) from which an elongate tubular cannula (304) extends distally. The cannula (304) includes a cannula wall (306) that defines a cannula lumen (308). A distal end (310) of the cannula (304) is beveled, including a long side (310a) substantially parallel with the central longitudinal axis of the cannula (304) and extending to its distal-most tip end. A short side (310b) of the beveled distal end (310) is opposite the long end (310a). A detailed illustration of the distal end (310) is shown in the top plan view of FIG. 3B. Other embodiments may include a double bevel, where one beveled surface is opposite the notch (320), or single or double bevels that are at least partially transverse relative to the notch (320). As will be further described below, any of the foregoing aspects of the presently disclosed biopsy needle may include one or more laser cut apertures disposed in the cannula wall near a distal portion or distal end (310) of the elongate tubular cannula.

Specific aspects of the present disclosure are shown in the side plan view of FIGS. 3A and 3C, and in the perspective view of FIG. 3D. Specifically, a notch (320) is disposed proximally adjacent to the beveled distal cannula end (310) and is generally centered in longitudinal alignment with the long beveled end side (310a) and opposite the short beveled end side (310b). In certain aspects, the notch (320) is generally arcuate, defined on its proximal side by a parabolic edge (322) extending along generally longitudinal, but somewhat curved lateral notch sides (324). In some aspects, the biopsy needle device (300) does not include a notch.

The distal edge (324) of the notch (320) may be formed as a generally parabolic lip that joins the proximal edge (322) at a pair of lip end portions (326) that may provide a curved transition between the proximal lateral and distal edges (322, 324). In certain aspects, the radiused lip end portions (326) are configured to provide stress relief within the cannula structure. A central distal lip portion (325) of the distal edge (324) may form a proximal-facing cutting edge. In certain aspects, the notch will occupy less than or about one-half the circumference of the cannula (304) at the broadest point of the notch.

Inclusion of the bevel illustrated in FIG. 3C may provide advantages for successful sample collection. Specifically, contact of the bevel face against tissue may create a slight bias / pressure toward the notch that will help tissue to be pulled / captured into the notch when the stylet (described below) is withdrawn, including that contact pressure on the notch side of the shaft surface may be slightly greater than on the exterior surface immediately opposite the notch. The cannula has a consistent or at least substantially consistent outer diameter along at least its distal length from distal of the notch, across the notch, and proximal of the notch. As will be further described below, any of the foregoing aspects of the presently disclosed biopsy needle disclosed in FIGS. 3A - 3D may include one or more apertures disposed in the cannula wall near a distal portion or distal end of the elongate tubular cannula.

The present disclosure is also intended to cover a needle having a notch at its distal end configured in a direction opposite to that depicted in FIGS. 3A - 3D. That is, as shown in the side plan view of FIGS. 2A and 2C, and in the perspective view of FIG. 2D, a notch (220) is disposed proximally adjacent to the beveled distal cannula end (210) and is generally centered in longitudinal alignment with the long beveled end side (210a) and opposite the short beveled end side (210b). In certain aspects, the notch (220) is generally arcuate, defined on its distal side by a parabolic edge (222) extending along generally longitudinal, but somewhat curved lateral notch sides (224). The proximal edge (224) of the notch (220) may be formed as generally parabolic lip that joins the distal edge (222) at a pair of lip end portions (226) that may provide a curved transition between the distal lateral and proximal edges (222, 224). The radiused lip end portions (226) may be configured to provide stress relief within the cannula structure. A central proximal lip portion (225) of the proximal edge (224) may form a distal-facing cutting edge. In certain aspects, the notch will occupy less than or about one-half the circumference of the cannula (204) at the broadest point of the notch.

Inclusion of the bevel illustrated in FIG. 2C may provide advantages for successful sample collection. Specifically, contact of the bevel face against tissue may create a slight bias/ pressure toward the notch that will help tissue to be pulled/ captured into the notch when the stylet (described below) is withdrawn, including that contact pressure on the notch side of the shaft surface may be slightly greater than on the exterior surface immediately opposite the notch. The cannula has a consistent or at least substantially consistent outer diameter along at least its distal length from distal of the notch, across the notch, and proximal of the notch. As will be further described below, any of the foregoing aspects of the presently disclosed biopsy needle disclosed in FIGS. 2A - 2D may include one or more apertures disposed in the cannula wall near a distal portion or distal end of the elongate tubular cannula.

As can be seen in FIGS. 2B and 2C, an elongate stylet (230) may be disposed removably through the cannula lumen (208). As can be seen in FIGS. 3A - 3D, an elongate stylet (330) may be disposed removably through the cannula lumen (308). In certain aspects, the stylet (230, 330) will occupy substantially or nearly an entire cross-sectional area of at least a lengthwise portion of the cannula lumen (208, 308). As shown in FIG. 2C, a distal end (232) of the stylet (230) may be rounded and dimensioned not to extend out of the distal beveled cannula end (210). This construction will provide enhanced support for the cannula (particularly during navigation to a target site). It should be appreciated that a beveled stylet end or other stylet end configuration may be practiced within the scope of the present disclosure and, in the case of a beveled-end stylet, may provide a generally solid cutting and/or tissue-penetrating distal tip end formed by matching bevels of the stylet (230, 330) and cannula (204, 304).

In certain aspects, the needle has an open distal end, but, in other aspects, the distal beveled needle end may be closed, such that the lumen (208, 308) extending longitudinally through the cannula terminates within the cannula (204, 304). In these aspects, a stylet may be reinserted into the needle lumen after the sample has been excised and captured through the notch into the needle lumen. In such a circumstance, the stylet may be extended distally to cover the open notch (thereby preventing contamination of the sample by inadvertent collection of cells along the needle track during withdrawal of the needle), but leaving room in a closed needle lumen portion for the sample to remain intact between the notch and a closed distal end in an embodiment where the needle lumen is closed at the distal end.

In one specific aspect, with general reference to FIGS. 2A-2D, the cannula (204) may be constructed as a 20-gauge needle made of 304 stainless steel, with an inner diameter of about 0.9 mm (about 0.03 inches) and an outer diameter of about 0.91 mm (about 0.036 inches). In this aspect, the notch (220) may be circumferentially located opposite and proximal of a distal bevel that is at about a 18.3° angle (±about 5°) relative to the short side such that a proximal-most end of the notch (220) (defined by the curved lip end portion (226)) is about 7.1 mm (about 0.28 inches) longitudinally proximal of the distal-most tip end of the cannula (204). In this aspect, the longitudinal distance between the distal-most notch edge (222) and the distal-most portion of the rounded proximal cutting lip (225) will be about 3 mm (about 0.12 inches). The distal-most portion of the proximal lip (225) will be about 0.56 mm (about 0.022 inches) from the proximal-most end of the notch (220), which will be defined by a curved lip end portion (226), including a radius of curvature of about 0.05 mm (about 0.002 inches), joining the proximal edge (224) with the distal edge (222). A beveled or round-tipped NiTi stylet (230) may be disposed slidably / removably through the cannula lumen. It should be appreciated that, while a 20-gauge needle is exemplified, larger gauge needles, such as 19-gauge, 18-gauge, 17-gauge, etc., or smaller gauge needles, such as 22-gauge, 23-gauge, 24-gauge, 25-gauge, etc., may be practiced within the scope of the present disclosure (although, it will be appreciated that their absolute dimensions will vary from those disclosed for the 20-gauge example). The notch may be oriented with a proximal-facing cutting lip (e.g., such as is disclosed in co-owned U.S. Pat. Publ. 2012/0253228 to Schembre et al.,). The needle may also include one or more apertures disposed in the cannula wall near a distal portion or distal end of the elongate tubular cannula.

In a second specific aspect, with general reference to FIGS. 3A-3D, the cannula (304) may be constructed as a 20-gauge needle made of 304 stainless steel, with an inner diameter of about 0.9 mm (about 0.03 inches). In this aspect, the notch (320) may be circumferentially located opposite and proximal of a distal bevel that is at about a 30° angle relative to the short side such that a proximal-most end of the notch (320) (defined by the proximal edge (322)) is about 9 mm (about 0.36 inches) longitudinally proximal of the distal-most tip end of the cannula (304). In this aspect, the longitudinal distance between the proximal-most notch edge (322) and the proximal-most portion of the distal lip (325) may be about 4 mm to about 5 mm. The proximal-most portion of the distal lip (325) may be about 0.6 mm (about 0.025 inches) from the distal-most end of the notch (320), which will be defined by a curved lip end portion (326), including a radius of curvature of about 0.05 mm (about 0.002 inches), joining the distal edge (324) with the proximal edge (322). The longitudinal linear distance between the distal-most lip end portions (326) and the proximal end of the bevel 310) opposite the notch (320) may be about 1.47 mm in 18-gauge, 19-gauge, 20-gauge, 21-gauge, or 22-gauge embodiments to provide optimal strength and notch position relative to the needle's distal end. A beveled or round-tipped NiTi stylet (330) may be disposed slidably / removably through the cannula lumen. It should be appreciated that, while a 20-gauge needle is exemplified, larger gauge needles, such as 19-gauge, 18-gauge, 17-gauge, etc., or smaller gauge needles, such as 22-gauge, 23-gauge, 24-gauge, 25-gauge, etc., may be practiced within the scope of the present disclosure (although, it will be appreciated that their absolute dimensions will vary from those disclosed for the 20-gauge example). The needle may also include one or more apertures disposed in the cannula wall near a distal portion or distal end of the elongate tubular cannula.

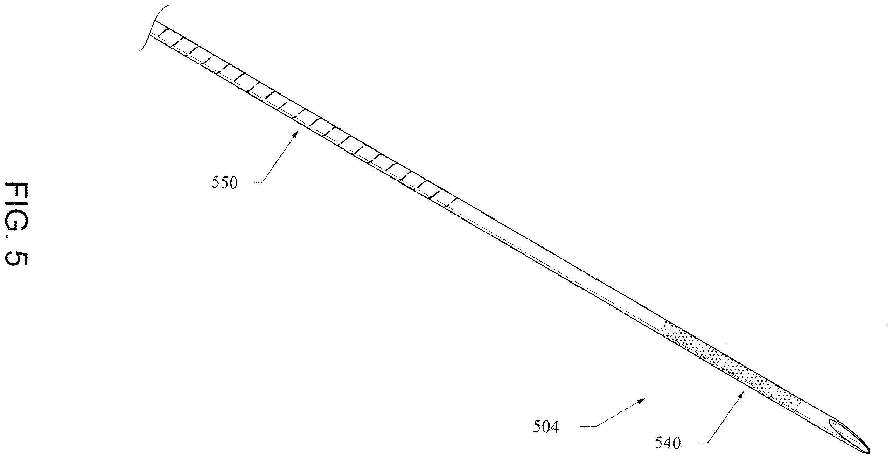

Although shown specifically in connection with the embodiment depicted in FIG. 2A and FIG. 5, any aspect of the presently disclosed needle may include surface features (240/540) configured to enhance echogenicity, thereby providing an improved ability to navigate the device during an EUS procedure. The surface features (240/540) are shown here as dimples on an exterior surface of the cannula (204 / 504), but may alternatively be embodied as grooves or other regular or irregular features on an external or internal surface. Embedded echogenic features such as bubbles, voids, or pieces of echo-contrasting materials may also be used within the scope of the present disclosure. Those of skill in the art will appreciate that many currently-known and/or future-developed echogenicity-enhancing means may be used within the scope of the present invention. As used herein, the terms echogenic and echogenicity-enhancing are used to refer to structural features that increase the reflectivity of ultrasound waves used during ultrasound visualization of a device, with the increase being over the typical ultrasound reflectivity/ visualizability of a device lacking the features described.

The echogenic features (240 / 540) may extend distally across the cannula surface radially opposite space occupied by the notch. In certain aspects, the echogenicity-enhancing features are disposed at a specified predetermined distance from the distal-most tip end of the cannula. Although the echogenic features (240) in FIG. 2A are shown at a distance from the notch, a cannula according to the present disclosure may be constructed with those echogenic features disposed flush up to the margins of the notch. The stylet (230, 330) may include echogenicity-enhancing features instead of, or in addition to, those that may be disposed on the cannula (204, 304).

If a sheath (170) is included with the tissue-sampling needle, any sheath known to those of skill in the art may be utilized in accordance with this disclosure. In certain aspects, the sheath (170) may include a coiled coated-wire tube defining a longitudinal sheath lumen as disclosed in U.S. Patent Application Ser. No. 14/195,333 titled "Endoscopic Biopsy Needle With Coil Sheath," filed on March 3, 2014, by Leahy *et* al. The needle cannula is slidably disposed through the sheath lumen and the sheath (170) is fixed to a portion of the handle (162) (see, for example, U.S. Patent Application Ser. No. 14/195,333).

The distal end of the device may be subjected to severe curvature(s) when directed through an endoscope working channel to a target site, particularly at exit, e.g. when flexed by a duodenoscope elevator to an approximate 90° longitudinal transition. Such severe curvature exerts forces on the cannula that could increase a risk of undesirable buckling and/or kinking, particularly in a distal portion of the cannula. To avoid any such deformation, buckling, kinking, etc., of the distal portion of the cannula, any aspect of the needles disclosed herein may comprise one or more apertures in a distal portion of the cannula to enhance flexibility.

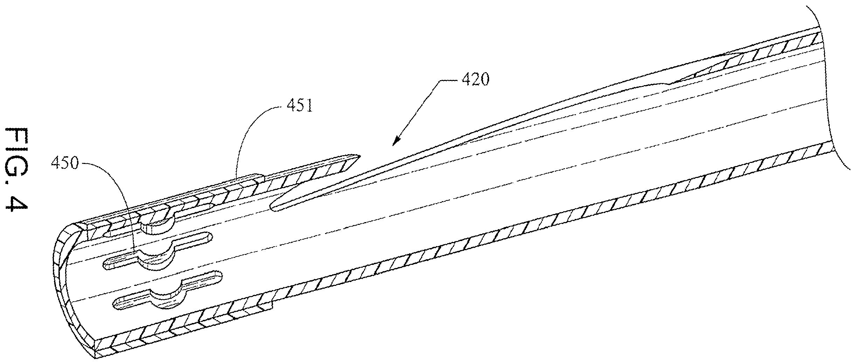

FIGS. 4-7 show aspects of the distal portion of the presently disclosed biopsy needle having apertures providing flexibility or enhanced flexibility (as compared to a needle lacking such apertures, but otherwise the same gauge, wall thickness, etc.). FIG. 4 shows an aspect of the presently disclosed biopsy needle comprising a notch (420) configured for capturing tissue samples (e.g. as described above). A longitudinal and transverse section view of the distal portion of the cannula can be seen including the notch (420) and a plurality of apertures (450). The plurality of apertures (450) adds flexibility to the cannula and/or distal portion of the cannula so that while navigating through a medical device, the cannula and/or distal portion of the cannula does not kink or buckle. The plurality of apertures may be covered by a sealing member, such as a wrap (451), as will be further described hereinbelow.

FIG. 5 shows another embodiment of the biopsy needle, which does not include a notch but, in certain embodiments, it may include a notch. The distal end of the cannula (504) shown in FIG. 5 includes surface features (540), configured to enhance echogenicity, thereby providing an improved ability to navigate the device during an EUS procedure. Proximal of the surface features (540) is a plurality of apertures (550). In this specific aspect, the apertures (550) are configured in an interrupted helical cut pattern. The interrupted helical cut patterns are offset on the cannula to allow the distal portion of the cannula to have flexibility in any direction, i.e. 360° of movement. If the apertures were parallel / lined up one directly above the next from a proximal end of the cannula to a distal end, the present inventors have discovered that the cannula would have limited movement in certain directions.

In connection with any of the presently disclosed needle embodiments, including the embodiments depicted in FIGS. 2A-2D and 3A-3D, the apertures on the cannula may span almost any length of the cannula. In some aspects, the cannula comprises from about 3 inches (about 7.6 cm) to about 8 inches (about 20.3 cm) of apertures disposed in its surface. In other aspects, the cannula comprises from about 4 inches (about 10.2 cm) to about 7 inches (about 17.8 cm) of apertures disposed in its surface. For example, in one aspect, the length from the distal tip of the cannula to the distal-most aperture may be from about 0.75 inches (about 1.9 cm) to about 1.5 inches (about 3.8 cm) or any sub-range thereof. According to the present invention, the length from the distal tip of the cannula to the distal-most aperture is from about 1 inch (about 2.5 cm) to about 1.25 inches (about 3.2 cm).

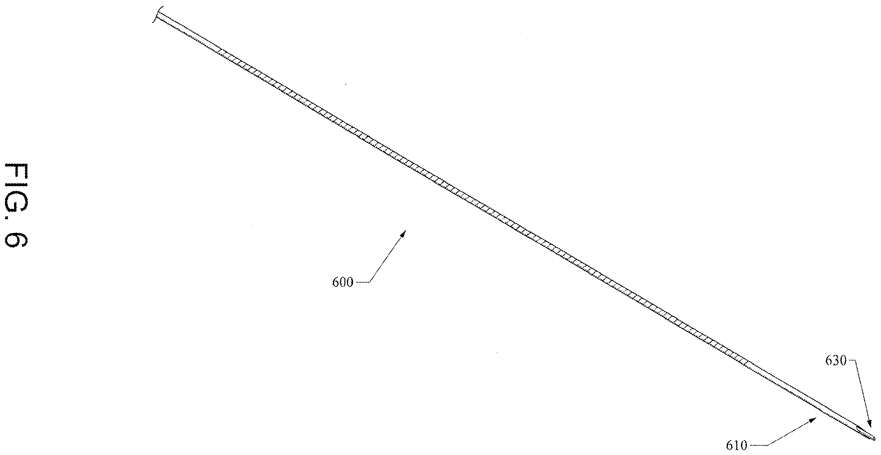

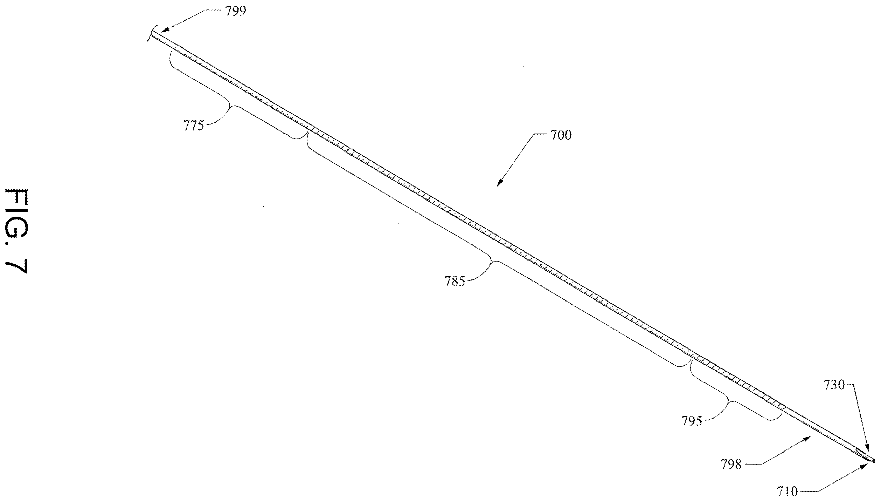

In turn, the length from the distal-most aperture to the proximal-most aperture may be from about 3 inches (about 7.6 cm) to about 8 inches (about 20.3 cm). According to the present invention, the distal portion of the cannula comprises about 5 inches (about 12.7 cm) in length of the apertures (see FIG. 6). In other aspects, the distal portion of the cannula comprises about 7 inches (about 17.8 cm) in length of the apertures (see FIG. 7).

FIG. 6 depicts a distal portion of a cannula (600) having a plurality of apertures (650) disposed therein. The apertures (650) span about 5 inches (about 12.7 cm) in length of the distal end of the cannula and are set back about 1.2 inches (about 3 cm) from the distal tip (610). A stylet (630) is shown protruding from the distal tip (610). Having about 5 inches (about 12.7 cm) in length of the apertures (650) allows this portion of the needle to be directed efficiently and effectively through a tortuous distal section of an endoscope up to and including out of an exit aperture and fully-actuated endoscope elevator. This feature provides a highly desirable flexibility to the needle. Having the distal-most aperture set back about 1.2 inches (about 3 cm) from the distal tip (610) allows the needle to have sufficient pushability to enter the target anatomy, which would not be achievable if the apertures extended to the distal tip (610). Moreover, having the apertures set back from about 0.75 inches (about 1.9 cm) to about 1.5 inches (about 3.8 cm) from the distal tip (610) allows the user to enhance the echogenicity of a sufficient length of the distal end of the needle not including the apertures.

FIG. 7 depicts a distal portion of a cannula (700) having a plurality of apertures disposed therein. The apertures span about 7 inches (about 17.8 cm) in length of the distal end of the cannula and are set back about 1 inch (about 2.54 cm) from the distal tip (710). A stylet (730) is shown protruding from the distal tip (710). Having about 7 inches (about 17.8 cm) in length of the apertures allows this portion of the needle to sit within a tortuously curved distal section of an endoscope and provide the desired flexibility to the needle through that section. With the apertures distributed along about 7 inches (about 17.8 cm) of the cannula length, the user of the device may advance the distal end of the cannula up to about 3.1 inches (about 7.9 cm) into the target anatomy. Having the distal-most aperture set back about 1 inch (about 2.54 cm) from the distal tip (710) allows the needle to have sufficient pushability to enter the target anatomy, which would not be achievable if the apertures extended to the distal tip (710) and made it too flexible. Moreover, having the apertures set back from about 0.75 inches (about 1.9 cm) to about 1.5 inches (about 3.8 cm) or more from the distal tip (710) allows the user to enhance the echogenicity of a sufficient length of the distal end of the cannula not including the apertures.

The distal portion of the cannula comprising the apertures in FIG. 7 is depicted with three sections (775, 785, and 795). In FIG. 7, the proximal section (775) is about 1.6 inches (about 4 cm) in length, the middle section (785) is about 4.4 inches (about 11.2 cm) in length, and the distal section (795) is about 1 inch (about 2.54 cm) in length, although these sections may have alternate lengths in other aspects of the present disclosure. The proximal section (775) and the distal section (795) comprising the apertures may be less flexible than the middle section (785). This configuration provides a gradual change in flexibility from a proximal portion (799) to a distal needle portion (798) of the cannula. That is, the proximal portion (799) may be relatively inflexible, the proximal section (775) may be more flexible compared to the proximal portion (799), the middle section (785) may be more flexible than the proximal section (775) and the distal section (795), and the distal cannula portion (798) may have less flexibility than the distal section (795). In some aspects, the flexibility of portions (798) and (799) is about equivalent and the flexibility of sections (775) and (795) is about equivalent, where section (785) has the highest degree of flexibility.

Different iterations of aperture patterns may be used in accordance with any aspect of the presently disclosed needle and/or cannula. As an illustrative example with respect to FIG. 7, interrupted helical patterns are depicted. Proximal section (775) may comprise apertures having a ratio from about 110°:40° to about 120°:30°, where, for example, 120°:30° is the ratio of the cut (aperture) to the uncut section, i.e. 120° of a cut channel, followed by 30° uncut, followed by 120° of a cut channel, followed by 30° uncut, etc. The ratio may gradually change from about 110°:40° to about 120°:30° moving distally through section (775). Section (785) may have a constant ratio of 120°:30° and then moving distally through section (795), the ratio may gradually change from about 120°:30° to about 110°:40°.

An appropriate ratio may be selected by the user and all such ratios are intended to be covered by the present disclosure. For example, a suitable ratio may be about 110°:50°, about 120°:40°, or about 210°:30°. Moreover, the ratio may gradually change, as described above, from about 110°:50° at a proximal most portion of the apertures, to about 120°:40° at a mid-section of the apertures, and back to about 110°:50° at a distal most portion of the apertures.

The laser making the cuts may be set at any pitch desired by the user, such as about 1 mm, about 2 mm, about 3 mm, and so on. In some embodiments, the length of the overall pattern may be set to any desired pitch and in some embodiments, each individual aperture may also have any desired pitch. In some aspects, the pitch may change over a length of the cannula. For example, with respect to FIG. 7, the pitch may start at about 2 mm at the proximal most portion of section (775) and gradually decrease to about 1 mm at the distal most portion of section (775). The pitch may remain at about 1 mm throughout section (785) and at the proximal most portion of section (795). Extending distally through section (795), the pitch may gradually increase from about 1 mm to about 2 mm at the distal most portion of section (795). Again, while a specific pitch or a specific ratio may have been described, any desirable pitch or ratio may be used in accordance with the present disclosure.

The overall pattern may be pitched such that the distance between each aperture in the interrupted helical configuration may be selected by the user to be any specific distance and further, the distance of uncut cannula between each aperture may be adjusted by the user such that the distance changes throughout the interrupted helical pattern. Alternatively, the uncut portions (distance between each aperture) may be the same between each aperture. The distance between each aperture may be selected by the user and the present disclosure is intended to cover any distance between each aperture. It should be understood, however, that in some embodiments, pushability or advancement of the needle may become a problem if the distances between each aperture are minimal.

For example, it was previously noted that in one embodiment, the interrupted helical pattern may comprise about 120° of a cut channel (aperture) followed by about 30° of an uncut portion, followed by about 120° of a cut channel (aperture), followed by about 30° of an uncut portion, and so on along the cannula shaft. If the uncut portion were reduced, for example, to about 5° between each cut portion, the cannula may become extremely flexible and lose sufficient pushability.

In additional exemplary embodiments, the pitch of each individual aperture may be in the range of about 0.1 mm to about 4 mm, such as from about 0.5 mm to about 3 mm or from about 0.8 mm to about 2 mm. In some embodiments, each individual aperture may be pitched to achieve an offset such that the apertures are not perpendicular to the long axis of the cannula.

Further, although specific aperture locations are depicted in FIGS. 4-7, the plurality of apertures may be placed anywhere along the length of the cannula body. In the embodiment shown in FIG. 4, the plurality of apertures (450) is placed in between the notch (420) and the distal end (not shown) of the cannula. With the plurality of apertures at this location, flexibility or enhanced flexibility is added to the cannula at a distal location with respect to the cannula cutting features, e.g. the notch (420). With this configuration, the needle is able to maintain the same striking force when taking biopsy samples. In other aspects (not shown), the plurality of apertures may be located proximally adjacent the notch (420). Moreover, as previously noted, certain aspects of the presently disclosed needle do not include a notch, such as those shown in FIGS. 5, 6, and 7, and in those aspects, the plurality of apertures may run proximally along the cannula body starting at a point from about 0.75 inches (about 1.9 cm) to about 1.5 inches (about 3.8 cm) from the distal tip of the cannula.

While the presently disclosed apertures may take many shapes, in FIG. 4, the apertures are shown as substantially straight lines cut parallel to the longitudinal axis of the cannula body having a curved or semi-circular mid-portion. That is, each of the plurality of apertures (450) comprises a linear pattern parallel to the longitudinal axis of the cannula with a semi-circular pattern substantially in the center thereof.

In other specific aspects, such as those shown in FIGS. 5-7, the apertures comprise an interrupted helical pattern. When any aspect of the presently disclosed biopsy needle comprises the presently disclosed apertures, the needle is able to maintain the same striking force when taking biopsy samples, even though portions of the cannula body have been cut away to form the apertures. Moreover, with respect to certain aperture shape designs, such as those in FIGS. 5-7, the distal portion of the cannula may achieve enhanced flexibility in any direction, i.e. 360° of enhanced flexibility. Again, other shapes of apertures are contemplated by the present disclosure such as, but not limited to, straight lines substantially perpendicular and/or substantially parallel to the longitudinal axis of the cannula (and/or straight lines positioned at any other angle with respect to the longitudinal axis of the cannula), "+" shapes, "-" shapes, "V" shapes,"{" shapes, "(" shapes, "<" shapes, "[", shapes, etc.

If the distal portion or distal end of a cannula were to buckle or kink, as it easily could in a needle being subjected to the tortuous pathways in an endoscope, for example, numerous problems may occur. For example, once the kink forms, the outer diameter of the cannula would increase and/or become modified, thereby possibly tearing any component surrounding or coming into contact with the kinked portion of the cannula. Also, a kink in the cannula could lead to difficulties when attempting to withdraw the stylet, when attempting to use suction, and/or when attempting to inject fluid, such as contrast fluid, through the cannula. However, by incorporating the presently disclosed apertures into the cannula, any aspect of the presently disclosed needle can avoid the problems associated with cannula kinking or buckling.

While the presently disclosed plurality of apertures is not limited to a particular number of apertures, flexibility may be enhanced with a higher number of apertures or by placing the apertures closer together, for example. However, striking force could be compromised if the flexibility is too great. Of course, the number of apertures or proximity of each aperture to the neighboring aperture also depends upon the size of the aperture, where a location along the cannula could comprise more apertures if the apertures are smaller in size. If desired, the apertures may be placed at more than one location along the cannula body. For example, one or more apertures may be placed between the distal end of the cannula and the notch, and one or more apertures may be placed at a proximal location with respect to the notch.

The apertures may be formed in the cannula body by many different known manufacturing methods. For example, the apertures may be laser cut into the cannula body or they may be formed using electrical discharge machining (EDM).

In any aspect of the present disclosure, the plurality of apertures may be covered by a sealing member, such as a wrap (see 451 in FIG. 4). By cutting apertures into the cannula wall, cannula suction can be compromised and possibly even lost. Therefore, in certain aspects of the present disclosure, a wrap (451) may encompass an external surface of the cannula wall where the apertures have been cut. The wrap (451) forms a seal over the plurality of apertures so that suction may be utilized, if necessary. Any location along the cannula body comprising the plurality of apertures may also comprise the wrap (451).

The wrap (451) is not limited to a particular material and may be applied to the cannula by many different known manufacturing means. In one aspect, the wrap may be a plastic tube, and a heat shrinking process may be used to mold the wrap over the apertures on the external surface of the cannula. In some aspects, the wrap may be applied to the external surface of the cannula using insert molding. In illustrative, non-limiting examples, the wrap may comprise a polyimide, parylene, polytetrafluoroethylene (PTFE), polyether ether ketone (Peek), fluorinated ethylene propylene (FEP), and any combination thereof. In some embodiments, the wrap may be formed by spray coating the needle surface with any of these materials or any other material that could be used to form a seal over the apertures.

In other aspects, the sealing member may be a lining. The lining may be applied on the inside of the cannula, i.e. on the wall of the inner diameter of the cannula lumen, to accomplish the same goal as the wrap (451). With a lining applied to an interior wall of the lumen, thereby forming an interior seal over the apertures, desired suction may be maintained. In illustrative, non-limiting examples, the lining may comprise a polyimide, parylene, PTFE, Peek, FEP, and any combination thereof.

Those of skill in the art will appreciate with general reference to FIGS. 2A-2D that a method of tissue collection may be implemented using any aspect of the biopsy needle described herein. In one aspect of the method, the needle cannula, with the stylet disposed therein, may be directed via the working channel of a surgical visualization device (e.g., an EUS duodenoscope) into a target site to be sampled (e.g., a suspected tumor mass in the head of a patient's pancreas). The stylet may be withdrawn and suction applied to the needle cannula lumen (e.g. at a low-level of suction by withdrawal of a stylet that contacts most or all of the cannula to form a vacuum during retraction, or by external means such as a syringe or other vacuum-providing structure). While the distal portion or distal end of the cannula has a plurality of apertures disposed therein, suction is still available to the user due to the wrap/lining forming a seal over the apertures as described above. One or more of suction, rotary manipulation, and/or longitudinal manipulation of the needle cannula will excise (e.g., via the cutting lip) and capture tissue, which preferably will include sufficiently intact samples for histology, from the target site through the notch into the cannula lumen.

In an alternate aspect, and with general reference to FIGS. 3A-3D, a method of tissue collection is disclosed wherein the needle cannula, with the stylet disposed therein, is directed into a target site to be sampled (e.g., a tumor mass). The stylet may be withdrawn and suction applied to the proximal end of the needle cannula lumen. While the distal portion or distal end of the cannula has a plurality of apertures disposed therein, suction is still available to the user due to the wrap/lining forming a seal over the apertures as described above. The suction will pull tissue from the target site through the notch and into the lumen of the cannula. The user of the device can then quickly retract the cannula proximally such that the proximal-facing cutting edge of the distal notch's central lip cuts a sample of tissue from the target site that is drawn into the lumen and that may be captured within, distal, or proximal to the notch. The cannula may be advanced and retracted slightly (e.g. about half a centimeter, two or three times) and/or rotated or otherwise manipulated by the user if desired to try to capture sample material. The cannula may be bowed slightly during use to accentuate contact of the notch with adjacent tissue to promote improved sample collection.

The sample obtained according to any of the methods disclosed herein preferably will include a desirable number of intact cells, and most preferably more intact cells than are ordinarily obtained using a non-notched FNA biopsy needle ("more" indicating both a greater number and a higher degree of tissue/cell integrity within the sample obtained). It has been found that histological-grade FNB samples may be obtained in this manner, which may be preferred for certain diagnostic purposes over the cytological-grade samples typically obtained through FNA. The needle may then be withdrawn from the patient's body.

According to certain aspects of the methods disclosed herein, the cannula may be directed through a working channel of a peroral endoscope, such as a duodenoscope, into a patient's body. The distal end of the cannula may then be navigated (under ultrasound visualization if echogenicity-enhancing features are provided) into a target site. In other aspects, the biopsy needle may be introduced through other access means known in the art. These means may include percutaneous means, such as direct insertion of the needle cannula through a patient's skin or insertion through a trocar, sheath, or other access device (with or without endoscopic or ultrasound visualization).

As previously noted, it should also be appreciated that an outer sheath may be disposed slidably along the exterior of the cannula and the needle retracted thereinto (and/or the sheath distally advanced) so that the sheath is disposed over the notch after the sample is collected. This configuration, which may be practiced within the scope of the present disclosure, may lessen the likelihood that the sample collected will become lost or contaminated during needle withdrawal.

### Experimental Examples

The presently disclosed needle/cannula comprising the plurality of apertures has greatly enhanced flexibility when compared to prior art needles not comprising the apertures.

Three point bend testing was carried out on a standard 19GA needle with no apertures and two 19GA needles comprising a plurality of apertures prepared in accordance with the present disclosure. For clarity, one of the needles comprising the apertures is referred to as "laser cut version 2" and the other needle comprising the apertures is referred to as "laser cut version 3." The length of each needle subjected to the three point bend tests was about 130 mm. Laser cut version 2 had a ratio of cut to uncut portions of 120°:30° and a pitch of 0.8 mm. Laser cut version 3 had a ratio of cut to uncut portions of 210°:30° and a pitch of 1 mm. The lumen of each needle also comprised the same standard stylet.

In a three point bend test, the amount of force required to deflect a material a set distance is measured. With respect to the uncut needle, the deflection force was found to be about 7.72 N. With respect to laser cut version 2, the deflection force was found to be about 3.16 N, which amounts to about a 59% increase in flexibility over the uncut needle. With respect to laser cut version 3, the deflection force was found to be about 2.81 N, which amounts to about a 64% increase in flexibility over the uncut needle. Therefore, the needles comprising apertures had much improved flexibilities when compared to a standard needle that does not comprise the plurality of apertures.

Those of skill in the art will appreciate that embodiments not expressly illustrated herein may be practiced within the scope of the present disclosure, including that features described herein for different embodiments may be combined with each other and/or with currently-known or future-developed technologies while remaining within the scope of the following claims. It is therefore intended that the foregoing detailed description be regarded as illustrative rather than limiting. Also, it should be understood that the following claims, including all equivalents, are intended to define the scope of this invention.

Any ranges given either in absolute terms or in approximate terms are intended to encompass both, and any definitions used herein are intended to be clarifying and not limiting. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges (including all fractional and whole values) subsumed therein.

## Claims

1. A needle (200) comprising:
an elongate tubular cannula (204) including a proximal portion, a distal portion, and a cannula wall (206) defining a cannula lumen (208), the cannula lumen extending longitudinally through the elongate tubular cannula,
the distal portion of the elongate tubular cannula comprising:
a distal end (210),
a section comprising a plurality of apertures (450) disposed through, and along a first length of, the cannula wall in a pattern that enhances flexibility relative to a second length of the cannula wall lacking the apertures, and a sealing member disposed upon the plurality of apertures, **characterized in that** the section comprising the plurality of apertures is about 12.7cm in length and comprises a distal-most aperture, wherein the distal-most aperture is located about 2.5cm from the distal end of the distal portion of the elongate tubular cannula.

2. The needle of claim 1, wherein the sealing member includes a wrap (451) encompassing an external surface of the cannula wall.

3. The needle of claim 1, wherein the sealing member includes a lining encompassing an internal surface of the cannula wall.

4. The needle of any one of claims 1-3, further comprising a stylet (630) disposed through, and occupying substantially an entire cross-sectional area of, at least a lengthwise portion of the cannula lumen.

5. The needle of claim 4, wherein a distal end of the stylet is beveled.

6. The needle of any one of claims 1-5, further comprising a pattern of echogenic surface features located between the distal end of the cannula and the plurality of apertures.

7. The needle of any one of claims 1-6, further comprising a sample-collection notch (420) through the cannula wall and open to the cannula lumen.

8. The needle of claim 7, wherein the sample-collection notch comprises a cutting edge.

9. The needle of any one of claims 1-8, wherein the plurality of apertures is disposed in an interrupted helical pattern around a circumference and along the first length of the elongate tubular cannula.

10. The needle of claim 9, wherein the interrupted helical pattern comprises a cut channel portion to uncut portion ratio in a range of about 110° to about 210° for the cut channel portion of cannula circumference to a range of about 30° to about 50° for the uncut channel portion of cannula circumference.

11. The needle of claim 10, wherein the ratio varies along the elongate tubular cannula from about 110°:40° at a proximal-most portion of the plurality of apertures to about 120°:30° near a mid-section of the plurality of apertures to about 110°:40° at a distal-most portion of the plurality of apertures.

12. The needle of claim 10, wherein the ratio varies along the elongate tubular cannula from about 110°:50° at a proximal-most portion of the plurality of apertures to about 120°:40° near a mid-section of the plurality of apertures to about 110°:50° at a distal-most portion of the plurality of apertures.

13. The needle of any one of claims 1-12, further comprising an elongate sheath (170), wherein the elongate sheath (170) comprises a longitudinal sheath lumen through which the needle is slidably disposed.

14. The needle of claim 1 or claim 2, in the form of a biopsy needle (300), comprising:
a notch through the cannula wall and open to the cannula lumen, and wherein the plurality of apertures is disposed proximal to the notch in an interrupted helical pattern.

15. The biopsy needle of claim 14, wherein the interrupted helical pattern comprises a cut channel portion to uncut portion ratio in a range of about 110° to about 210° for the cut channel portion of cannula circumference to a range of about 30° to about 50° for the uncut channel portion of cannula circumference.

## Patentansprüche

1. Nadel (200), umfassend:
eine längliche röhrenförmige Kanüle (204), die einen proximalen Abschnitt, einen distalen Abschnitt und eine Kanülenwand (206) aufweist, die ein Kanülenlumen (208) definiert, wobei sich das Kanülenlumen in Längsrichtung durch die längliche röhrenförmige Kanüle erstreckt,
wobei der distale Abschnitt der länglichen röhrenförmigen Kanüle, Folgendes umfasst:
ein distales Ende (210),
einen Abschnitt, der eine Vielzahl von Öffnungen (450) umfasst, die in einem Muster durch die Kanülenwand hindurch und entlang einer ersten Länge davon angeordnet sind, das die Flexibilität bezüglich einer zweiten Länge der Kanülenwand ohne die Öffnungen verbessert, sowie ein Dichtglied, das auf der Vielzahl von Öffnungen angeordnet ist,
**dadurch gekennzeichnet, dass** der die Vielzahl von Öffnungen umfassende Abschnitt ungefähr 12,7 cm lang ist und eine am weitesten distal liegende Öffnung umfasst, wobei die am weitesten distal liegende Öffnung ungefähr 2,5 cm vom distalen Ende des distalen Abschnitts der länglichen röhrenförmigen Kanüle angeordnet ist.

2. Nadel nach Anspruch 1, wobei das Dichtglied eine Umschlingung (451) aufweist, die eine äußere Fläche der Kanülenwand umschließt.

3. Nadel nach Anspruch 1, wobei das Dichtglied eine Verkleidung aufweist, die eine innere Fläche der Kanülenwand umschließt.

4. Nadel nach einem der Ansprüche 1 - 3, ferner umfassend ein Stilett (630), das durch mindestens einen Längsabschnitt des Kanülenlumens angeordnet ist und im Wesentlichen einen gesamten Querschnittsbereich davon einnimmt.

5. Nadel nach Anspruch 4, wobei ein distales Ende des Stiletts abgeschrägt ist.

6. Nadel nach einem der Ansprüche 1 - 5, ferner umfassend ein Muster aus echogenen Oberflächenmerkmalen, das zwischen dem distalen Ende der Kanüle und der Vielzahl von Öffnungen angeordnet ist.

7. Nadel nach einem der Ansprüche 1 - 6, ferner umfassend eine Probennahmekerbe (420), die durch die Kanülenwand geht und zum Kanülenlumen offen ist.

8. Nadel nach Anspruch 7, wobei die Probennahmekerbe eine Schneidkante umfasst.

9. Nadel nach einem der Ansprüche 1 - 8, wobei die Vielzahl von Öffnungen in einem unterbrochenen spiralförmigen Muster um einen Umfang herum und entlang der ersten Länge der länglichen röhrenförmigen Kanüle angeordnet sind.

10. Nadel nach Anspruch 9, wobei das unterbrochene spiralförmige Muster ein Verhältnis von geschnittenem Kanalabschnitt zu nicht geschnittenem Kanalabschnitt in einem Bereich von ungefähr 110° bis ungefähr 210° für den geschnittenen Kanalabschnitt des Kanülenumfangs zu einem Bereich von ungefähr 30° bis ungefähr 50° für den nicht geschnittenen Kanalabschnitt des Kanülenumfangs umfasst.

11. Nadel nach Anspruch 10, wobei das Verhältnis entlang der länglichen röhrenförmigen Kanüle von ungefähr 110°:40° an einem am weitesten proximal gelegenen Abschnitt der Vielzahl von Öffnungen bis zu ungefähr 120°:30° in der Nähe eines mittleren Abschnitts der Vielzahl von Öffnungen bis zu ungefähr 110°:40° an einem am weitesten distal gelegenen Abschnitt der Vielzahl von Öffnungen variiert.

12. Nadel nach Anspruch 10, wobei das Verhältnis entlang der länglichen röhrenförmigen Kanüle von ungefähr 110°:50° an einem am weitesten proximal gelegenen Abschnitt der Vielzahl von Öffnungen bis zu ungefähr 120°:40° in der Nähe eines mittleren Abschnitts der Vielzahl von Öffnungen bis zu ungefähr 110°:50° an einem am weitesten distal gelegenen Abschnitt der Vielzahl von Öffnungen variiert.

13. Nadel nach einem der Ansprüche 1 - 12, ferner umfassend eine längliche Hülse (170), wobei die längliche Hülse (170) ein längliches Hülsenlumen umfasst, durch das die Nadel verschiebbar angeordnet ist.

14. Nadel nach Anspruch 1 oder Anspruch 2 in der Form einer Biopsienadel (300), umfassend:
eine Kerbe, die durch die Kanülenwand geht und zum Kanülenlumen offen ist, und wobei die Vielzahl von Öffnungen proximal zu der Kerbe in einem unterbrochenen spiralförmigen Muster angeordnet sind.

15. Biopsienadel nach Anspruch 14, wobei das unterbrochene spiralförmige Muster ein Verhältnis von geschnittenem Kanalabschnitt zu nicht geschnittenem Kanalabschnitt in einem Bereich von ungefähr 110° bis ungefähr 210° für den geschnittenen Kanalabschnitt des Kanülenumfangs zu einem Bereich von ungefähr 30° bis ungefähr 50° für den nicht geschnittenen Kanalabschnitt des Kanülenumfangs umfasst.

## Revendications

1. Aiguille (200) comprenant une canule (204) tubulaire allongée comprenant :
une partie proximale,
une partie distale et
une paroi (206) de canule délimitant une lumière (208) de canule, la lumière de canule s'étendant longitudinalement dans toute la canule tubulaire allongée,
la partie distale de la canule tubulaire allongée comprenant :
une extrémité distale (210),
une section comprenant une pluralité d'ouvertures (450) disposées à travers la paroi de canule et le long d'une première longueur de celle-là, selon une disposition qui améliore la flexibilité par rapport à une seconde longueur de la paroi de canule dépourvue de ces ouvertures, et
un élément d'étanchéité disposé sur la pluralité d'ouvertures,
**caractérisé en ce que** la section comprenant la pluralité d'ouvertures a une longueur d'environ 12,7 cm et comprend une ouverture la plus distale,
l'ouverture la plus distale étant située à environ 2,5 cm de l'extrémité distale de la partie distale de la canule tubulaire allongée.

2. Aiguille selon la revendication 1, dans laquelle l'élément d'étanchéité comprend une enveloppe (451) entourant la surface externe de la paroi de canule.

3. Aiguille selon la revendication 1, dans laquelle l'élément d'étanchéité comprend un revêtement garnissant la surface interne de la paroi de canule.

4. Aiguille selon l'une quelconque des revendications 1 à 3, comprenant en outre un stylet (630) disposé à travers au moins une partie longitudinale de la lumière de canule et occupant sensiblement la totalité de sa section transversale.

5. Aiguille selon la revendication 4, dans laquelle l'extrémité distale du stylet est biseautée.

6. Aiguille selon l'une quelconque des revendications 1 à 5, comprenant en outre une configuration d'éléments de surface échogènes situés entre l'extrémité distale de la canule et la pluralité d'ouvertures.

7. Aiguille selon l'une quelconque des revendications 1 à 6, comprenant en outre une encoche (420) de prélèvement d'échantillon traversant la paroi de canule et s'ouvrant vers la lumière de canule.

8. Aiguille selon la revendication 7, dans laquelle l'encoche de prélèvement d'échantillon comprend un bord coupant.

9. Aiguille selon l'une quelconque des revendications 1 à 8, dans laquelle la pluralité d'ouvertures est disposée selon un motif hélicoïdal interrompu autour de la circonférence et le long de la première longueur de la canule tubulaire allongée.

10. Aiguille selon la revendication 9, dans laquelle le motif hélicoïdal interrompu comprend un rapport entre la partie découpée de canal et la partie non découpée allant d'une plage comprise entre environ 110° et environ 210° pour la partie découpée de canal de la circonférence de la canule à une plage comprise entre environ 30° et environ 50° pour la partie non découpée de canal de la circonférence de la canule.

11. Aiguille selon la revendication 10, dans laquelle le rapport varie le long de la canule tubulaire allongée d'environ 110°:40° au niveau de la partie la plus proximale de la pluralité d'ouvertures à environ 120°:30° près de la partie centrale de la pluralité d'ouvertures et à environ 110°:40° au niveau de la partie la plus distale de la pluralité d'ouvertures.

12. Aiguille selon la revendication 10, dans laquelle le rapport varie le long de la canule tubulaire allongée d'environ 110°:50° au niveau de la partie la plus proximale de la pluralité d'ouvertures à environ 120°:40° près de la partie centrale de la pluralité d'ouvertures et à environ 110°:50° au niveau de la partie la plus distale de la pluralité d'ouvertures.

13. Aiguille selon l'une quelconque des revendications 1 à 12, comprenant en outre une gaine (170) allongée, dans laquelle la gaine (170) allongée comprend une lumière longitudinale de gaine à travers laquelle l'aiguille est disposée coulissante.

14. Aiguille selon la revendication 1 ou 2, se présentant sous la forme d'une aiguille de biopsie (300), comprenant une encoche traversant la paroi de canule et s'ouvrant vers la lumière de canule, et
dans laquelle la pluralité d'ouvertures est disposée à proximité de l'encoche selon un motif hélicoïdal interrompu.

15. Aiguille de biopsie selon la revendication 14, dans laquelle le motif hélicoïdal interrompu comprend un rapport entre la partie découpée de canal et la partie non découpée allant d'une plage comprise entre environ 110° et environ 210° pour la partie découpée de canal de la circonférence de la canule à une plage comprise entre environ 30° et environ 50° pour la partie non découpée de canal de la circonférence de la canule.
